(19) Europäisches Patentamt
European Patent Office
Office européen des brevets

(11) Publication number: 0 245 991
A2

(12) # EUROPEAN PATENT APPLICATION

(21) Application number: 87303745.1

(22) Date of filing: 28.04.87

(51) Int.Cl.³: C 07 D 277/68
C 07 D 277/70, C 07 D 277/8-
2
C 07 D 417/12, A 01 N 43/78

(30) Priority: 13.05.86 GB 8611606

(43) Date of publication of application:
19.11.87 Bulletin 87/47

(84) Designated Contracting States:
AT BE CH DE ES FR GB GR IT LI LU NL SE

(71) Applicant: SCHERING AGROCHEMICALS LIMITED

Hauxton, Cambridge CB2 5HU(GB)

(72) Inventor: Mellor, Michael
2 Biscay Close
Haverhill Suffolk(GB)

(72) Inventor: Steele, Christopher Richard
4 Twyford Road
Bishops Stortford Herts(GB)

(74) Representative: Waldman, Ralph David et al,
Schering Agrochemicals Limited Industrial Property
Department Chesterford Park Research Station
Saffron Walden Essex CB10 1XL(GB)

(54) **Benzothiazoline fungicides.**

(57) A compound of formula I

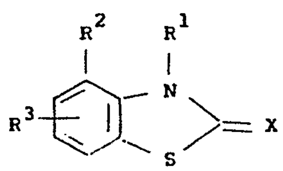

(I)

where $R^1$ is alkyl, alkenyl or alkynyl, each of which may be optionally substituted by halogen, alkoxy or aryl, $-N=CR^4R^5$ or $-NR^6R^7$;

$R^2$ is halo, hydroxy, amino, nitro, cyano, carboxy, alkoxycarbonyl or an optionally substituted alkyl, alkoxy, alkylthio, alkylamino, dialkylamino or alkylsulphonyl group;

$R^3$ is $-YR^9$;

$R^4$, $R^5$, $R^6$, $R^7$ and $R^8$ may be the same or different and are hydrogen, aryl or an optionally substituted alkyl, alkenyl or alkynyl group

$R^9$ has the same meaning as $R^8$ or is acyl;

X and Y are O, S or $NR^9$;

and when $R^1$ is $-N=CR^4R^5$ or $-NR^6R^7$, $R^3$ can also be hydrogen. The compounds have valuable fungicidal activity, especially against rice blast.

EP 0 245 991 A2

0245991

Case 86/11606

Benzothiazoline Fungicides

This invention relates to compounds having fungicidal activity.

Benzothiazolin(thi)one compounds having fungicidal activity are described in UK Patent 1564182. The compounds in this patent only carry one substituent on the benzo ring. We have now found that benzothiazolin(thi)one compounds, which are disubstituted in the benzo ring or which have an amino group in the 3-position, have valuable fungicidal properties.

According to the invention there is provided a compound of formula I

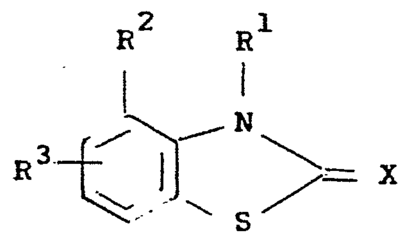

(I)

where $R^1$ is alkyl, alkenyl or alkynyl, each of which may be optionally substituted by halogen, alkoxy or aryl, $-N=CR^4R^5$ or $-NR^6R^7$;

$R^2$ is halo, hydroxy, amino, nitro, cyano, carboxy, alkoxycarbonyl or an optionally substituted alkyl, alkoxy, alkylthio, alkylamino, dialkylamino or alkylsulphonyl group;

$R^3$ is $-YR^9$;

$R^4$, $R^5$, $R^6$, $R^7$ and $R^8$ may be the same or

different and are hydrogen, aryl or an optionally substituted alkyl, alkenyl or alkynyl group

$R^9$ has the same meaning as $R^8$ or is acyl;

X and Y are O, S or $NR^9$;

and when $R^1$ is $-N=CR^4R^5$ or $-NR^6R^7$, $R^3$ can also be hydrogen.

Alkyl and alkoxy groups are preferably of 1 to 4 carbon atoms, especially methyl, and alkenyl and alkynyl groups are usually of 3 to 4 carbon atoms. Where the substitution is not defined, substituents, when present on any alkyl, alkoxy, alkenyl or alkynyl group, include halogen, alkoxy (e.g. of 1 to 4 carbon atoms), alkylthio, cyano, nitro, optionally substitiuted amino, carboxy, alkoxycarbonyl, acyloxy and aryl. Aryl groups are usually phenyl, optionally substituted, e.g. by halogen, alkyl, haloalkyl, alkoxy or nitro. The term aryl may include heteroaryl groups such as thienyl, furyl or pyridyl. The term 'acyl' includes the residue of sulphonic and phosphorus containing acids as well as carboxylic acids. Acyl groups are preferably alkanoyl e.g. of 1 to 4 carbon atoms. Amino groups may be substituted, e.g. by one or two alkyl or acyl groups or two substituents can form a ring, e.g. to form a morpholino or piperidino ring.

Preferably $R^3$ is in the 7-position, i.e para to $R^2$. In a particularly preferred group of compounds,

$R^1$ is alkyl, especially methyl, $R^2$ is halogen, especially chlorine, $R^3$ is alkoxy, especially methoxy, optionally substituted by alkoxy and X is oxygen.

The compounds of the invention have activity as fungicides, especially against fungal diseases of plants, and especially diseases of rice such as rice blast (Pyricularia oryzae). Other diseases against which the compounds may be active include mildews and particularly barley powdery mildew (Erysiphe graminis) and vine downy mildew (Plasmopora viticola) and blight of potatoes or tomatoes (Phytophthora infestans).

The invention thus also provides a method of combating fungi at a locus infested or liable to be infested therewith, which comprises applying to the locus a compound of formula I.

The invention also provides an agricultural composition comprising a compound of formula I in admixture with an agronomically acceptable diluent or carrier.

The composition of the invention may of course include more than one compound of the invention.

In addition the composition can comprise one or more additional active ingredients, for example compounds known to possess plant-growth regulant, herbicidal, fungicidal, insecticidal or acaricidal properties. Alternatively the

compounds of the invention can be used in sequence with the other active ingredient.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty

alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, a dispersion, an aqueous emulsion, a dusting powder, a seed dressing, a fumigant, a smoke, a dispersible powder, an emulsifiable concentrate or granules. Moreover it can be in a suitable form for direct application or as a concentrate or primary composition which requires dilution with a suitable quantity of water or other diluent before application.

An emulsifiable concentrate comprises a compound of

the invention dissolved in a water-immiscible solvent which is formed into an emulsion with water in the presence of an emulsifying agent.

A dusting powder comprises a compound of the invention intimately mixed and ground with a solid pulverulent diluent, for example, kaolin.

A granular solid comprises a compound of the invention associated with similar diluents to those which may be employed in dusting powders, but the mixture is granulated by known methods. Alternatively it comprises the active ingredient absorbed or adsorbed on a pre-granular diluent, for example, Fuller's earth, attapulgite or limestone grit.

Wettable powders, granules or grains usually comprise the active ingredient in admixture with a suitable surfactant and an inert powder diluent such as china clay.

Another suitable concentrate is a flowable suspension concentrate which is formed by grinding the compound with water or other liquid, a wetting agent and a suspending agent.

The concentration of the active ingredient in the composition of the present invention, as applied to plants is preferably within the range of 0.01 to 3.0 per cent by weight, especially 0.01 to 1.0 per cent by weight. In a primary composition the amount of active ingredient can vary widely and can be, for example, from 5 to 95 per cent

by weight of the composition.

In the method of the invention the compound is generally applied to seeds, plants or their habitat. Thus, the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable applications rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as protective measure. In both such cases the preferred mode of application is by foliar spraying. It is generally important to obtain good control of fungi in the early stages of plant growth as this is the time when the plant can be most severely damaged. The spray or dust can conveniently contain a pre- or post-emergence herbicide if

this is thought necessary. Sometimes, it is practicable to treat the roots of a plant before or during planting, for example, by dipping the roots in a suitable liquid or solid composition. When the active compound is applied directly to the plant a suitable rate of application is from 0.01 to 10 kg. per hectare, preferably from 0.05 to 5 kg per hectare.

The compounds of the invention may be prepared in a variety of ways. When $R^1$ is an alkyl, alkenyl or alkynyl group, these may be obtained by reacting a compound of formula II

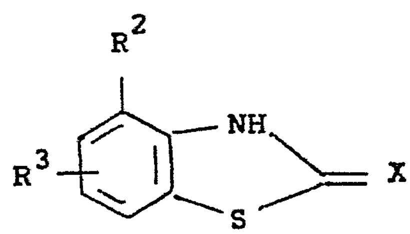

(II)

with a compound $R^1Q$, where Q is a leaving group, such as halogen and especially iodine, under basic conditions. The compounds of formula II, in which X is oxygen, can be prepared by hydrolysis of a compound of formula III

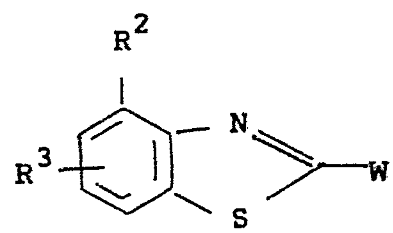

(III)

where W is a halogen group. Compounds of formula III can be obtained in known manner as for example by methods

described in British Patent 1,397,089.

Compounds of formula I where X is an imino group can be otained from a compound of formula III where W is amino by reaction with a compound of formula $R^1Q$, where Q is as defined above. The imino group can then be substituted in known manner e.g. by treatment with a compound of formula $R^9Q$. where Q is a defined above.

Compounds of formula I where $R^1$ is a substituted amino group can be obtained by known methods from a compound of formula IV

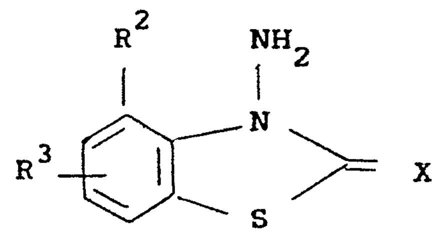

(IV)

The compound of formula IV may be obtained by reacting a substituted hydroxylamine with a compound of formula V

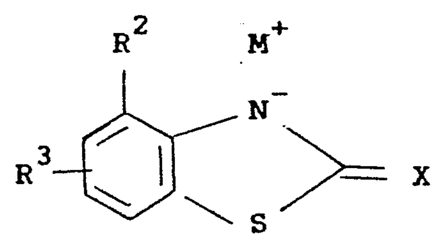

(V)

where M is a metal, e.g. potassium.

Compounds where $R^3$ is hydroxy may be obtained by demethylation of compounds where $R^3$ is alkoxy such as methoxy. The hydroxy compound may then be converted to other $R^3$ groups.

Compounds where X is sulphur can be obtained from compounds where X is oxygen by treatment with a sulphurising agent, e.g. Lawesson's reagent.

The invention is illustrated in the following Examples. Structures of isolated novel compounds were confirmed by elemental and/or other appropriate analyses. Temperatures are in °C.

Example 1

O-(2,4-Dinitrophenyl)hydroxylamine (26.17 g) was added to a solution of the potassium salt of 4-chloro-2-benzothiazolinone (29.4 g) in N,N-dimethylformamide (100 ml) and the mixture allowed to stand for 30 minutes with occasional swirling. After pouring into water (1700 ml), the yellow precipitate produced was collected by filtration, washed with water and then dissolved in dichloromethane (800 ml). The resultant solution was dried and evaporated. The crude product was recrystallised from toluene, following treatment with decolourising charcoal to give 3-amino-4-chloro-2(3H)-benzothiazolone, m.p. 166-167.5°. (Compound 1).

Example 2

A mixture of the product of Example 1 (4.0 g) with acetaldehyde in ethanol (40 ml) was refluxed for 5 minutes. Evaporation gave a crude product, which was purified by a combination of flash chromatography and

recystallisation (propan-2-ol) to give 4-chloro-3-ethylidenamino-2(3H)-benzothiazolone, m.p. 98-9°C. (Compound 2).

Example 3

Benzoyl chloride (12.12 g) was added dropwise to a stirred solution of ammonium thiocyanate (7.25 g) in dry acetone (50 ml). After stirring for 15 minutes, a solution of 2-chloro-3-methoxyaniline (13.6 g) in dry acetone (20 ml) was added dropwise with vigorous stirring. After stirring at room temperature for a further 30 minutes, the reaction mixture was refluxed for 5 minutes and then poured into water (500 ml). The solid produced was collected by filtration, washed with water and air-dried before dissolving in 10% aqueous sodium hydroxide (52 ml) and heating on a steam bath for 45 minutes. After dilution with water (100 ml), the pH was adjusted by first acidifying to pH 5 and then basifying to pH 9 with aqueous ammonia (S.G. = 0.88). The solid obtained was collected by filtration, washed with water and dried *in vacuo* to give crude N-(2-chloro-3-methoxyphenyl)thiourea which was used without further purification. A small sample was filtered through a bed of silica gel to give a pure sample for analysis, m.p. 201-204°.

Bromine (17.05 g) was added, dropwise to a stirred

suspension of the crude product (15.14 g) in acetic acid (13.5 ml) and 1,2-dichloroethane (55 ml). The resulting solution was refluxed for 5 hours. After cooling, the reaction mixture was diluted with diethyl ether and the precipitated solid collected by filtration, washed with diethyl ether and dried in vacuo to give the intermediate, 2-amino-4-chloro-5-methoxybenzothiazole hydrobromide (22.35 g). This salt was resuspended in acetic acid (13.5 ml) and 1,2-dichloroethane (55 ml) and after treating with concentrated hydrochloric acid (32 ml) and water (32 ml) was cooled to 0°. Sodium nitrite (12.71 g) in water (32 ml) was then added, portionwise, to the stirred mixture whilst maintaining a reaction mixture temperature of 5°. After stirring for a further 30 minutes at 0-5° and 90 minutes at room temperature, the reaction mixture was filtered. The organic phase of the filtrate was separated, washed with water, dried, filtered through a bed of silica gel and evaporated to give crude 2,4-dichloro-5-methoxybenzothiazole which was used without further purification. A mixture of this crude product (10.78 g), concentrated sulphuric acid (25 ml) and methanol (180 ml) was stirred at reflux for 5 hours. After cooling, the reaction mixture was poured into water (700 ml) and the solid produced, collected by filtration, washed well with water and dried in vacuo to give crude

4-chloro-5-methoxy-2(3H)-benzothiazolone. The product was used without further purification. A mixture of this crude product (3.0 g); and anhydrous potassium carbonate (1.92 g) in 2-butanone (50 ml) was stirred at reflux for 1 hour. Iodomethane (3.95 g) was then added and the reaction mixture stirred at reflux for a further 3 hours. After cooling, the reaction mixture was filtered and the filtrate evaporated. The residue was dissolved in dichloromethane filtered through a bed of silica gel and then evaporated. The residue was subjected to flash chromatography and then recrystallised from light petroleum/dichloromethane (2:1 mixture) to give 4-chloro-5-methoxy-3-methyl-2(3H)-benzothiazolone, m.p. 146-7° (Compound 3)

Example 4

A solution of sodium nitrite (17.1 g) in water (45 ml) was added dropwise to a stirred suspension of 2-amino-4-chloro-7-methoxybenzothiazole (14.88 g) in acetic acid (14 ml), 1,2-dichloroethane (60 ml), concentrated hydrochloric acid (45 ml) and water (45 ml) maintained at 0-5°. The mixture was stirred at 0° for one hour and then at room temperature for 3 hours. After filtration of the reaction mixture, the organic phase of the filtrate was separated, washed with water and filtered through silica gel. The solvent was evaporated and the



BAD ORIGINAL

residue redissolved in a mixture of light petroleum and dichloromethane and the solution filtered through silica gel. Solvent was evaporated to give crude 2,4-dichloro-7-methoxybenzothiazole.

A mixture of this crude product (38.0 g), concentrated sulphuric acid (72 ml) and methanol (528 ml) was stirred at reflux for 5 hours. After cooling, the reaction mixture was poured into water (1000 ml) and the solid produced, collected by filtration, washed well with water and dried _in vacuo_ to give crude 4-chloro-7-methoxy-2(3_H_)-benzothiazolone. The product was used without further purification. A mixture of this crude product (20.34 g); and anhydrous potassium carbonate (13.01 g) in 2-butanone (250 ml) was stirred at reflux for 1 hour. Iodomethane (14.7 g) was then added and the reaction mixture stirred at reflux for a further 3 hours. After cooling, the reaction mixture was filtered and the filtrate evaporated. The residue was recrystallised from cyclohexane to give 4-chloro-7-methoxy-3-methyl-2(3_H_)-benzothiazolone, m.p. 128-30° (Compound 4)

Example 5

Compound 4 (7.6 g) was heated under reflux for 12 hours with hydrobromic acid (200 ml of 48% solution). The solid was filtered off, washed with water and dried to give crude 4-chloro-7-hydroxy-3-methyl-2(3H)-benzothiazolone. (Compound 5)

Example 6

A mixture of compound 5 (1.1 g) and potassium carbonate (0.7 g) in 2-butanone was heated under reflux for 1 hour. Ethyl iodide (0.8 g) was added and the mixture heated under reflux for a further 3 hours. It was then cooled, filtered and evaporated and the residue purified by chromatography, followed by recrystallisation from cyclohexane to give 4-chloro-7-ethoxy-3-methyl-2(3H)-benzothiazolone, m.p. 122-4°. (Compound 6)

Example 7

A mixture of compound 4 (4.3 g) and Lawesson's reagent (11.5 g) in toluene (120 ml) was heated under reflux for 4 days. The liquid was decanted, cooled and the solid recrystallised from 2-butanone to give 4-chloro-7-methoxy-3-methyl-2(3H)-benzothiazolethione, m.p. 122-4°. (Compound 7)

0245991

Example 8

A mixture of 4-chloro-7-methoxy-2(3H)-benzothiazolinone (2.16 g) and potassium carbonate (1.38 g) in 2-butanone (20 ml) was heated under reflux for 1 hour. Ethyl iodide (1.56 g) was added and the mixture heated under reflux for a further 3 hours. It was then cooled, filtered and evaporated and the residue purified by chromatography to give 4-chloro-7-methoxy-3-ethyl-2(3H)-benzothiazolone, m.p. 115-7°. (Compound 8)

Example 9

In a similar manner to the method described in the previous Examples the following compounds were obtained

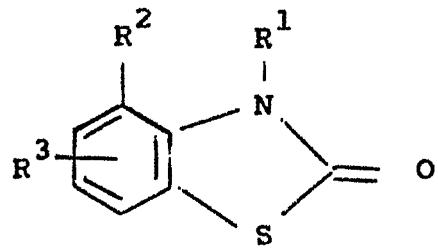

| Cpd No | $R^1$ | $R^2$ | $R^3$ | m.p. |
|---|---|---|---|---|
| 9 | Me | Cl | 6-MeO | 130-1 |
| 10 | MeCH=N | Me | H | 95-7 |
| 11 | PrCH=N | Cl | H | 51-2 |
| 12 | $NH_2$ | Br | H | 168-172 |
| 13 | $Me_2NCH_2NH$ | Cl | H | 134-7 |
| 14 | EtCH=N | Cl | H | 54-7 |
| 15 | $EtOCH_2NH$ | Cl | H | 100-2 |
| 16 | $MeOCH_2NH$ | Cl | H | 95 |
| 17 | Me | MeO | 7-MeO | 104-5 |
| 18 | Me | Me | 7-MeO | 92-3 |
| 18 | Me | Cl | 7-PrO | 92-4 |
| 20 | Me | Cl | 7-$Pr^iO$ | 84-6 |
| 21 | Me | Cl | 7-$PhCH_2O$ | 119-21 |
| 22 | Me | Cl | 7-MeCOO | 154 |
| 23 | Me | Cl | 7-$MeO(CH_2)_2O$ | 108-10 |

| Cpd No | $R^1$ | $R^2$ | $R^3$ | m.p. |
|---|---|---|---|---|
| 24 | Me | Cl | 7-$NCCH_2O$ | 150-2 |
| 25 | Me | Br | 7-EtO | 117.5-8.5 |
| 26 | Me | Cl | 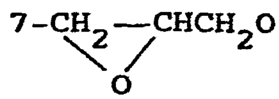 | 126-7 |
| 27 | Me | Br | 7-$MeO(CH_2)_2O$ | 55-60 |
| 28 | Me | Cl | 7-$MeS(CH_2)_2O$ | 110-1 |
| 29 | Me | Me | 7-$MeO(CH_2)_2O$ | 86-8 |
| 30 | Me | $PhCH_2O$ | 7-MeO | 151.5-152.5 |
| 31 | Me | Cl | 7-$CH{\equiv}CCH_2O$ | 142-143.5 |
| 32 | Me | Br | 7-$NCCH_2O$ | 194-196.5 |
| 33 | Me | Cl | 7-$CH_2{=}CHCH_2O$ | 92-3 |
| 34 | Me | Me | 7-$NCCH_2O$ | 163-4 |
| 35 | Me | F | 7-MeO | 104-9 |
| 36 | $NH_2$ | Cl | 7-MeO | 92-3 |

Example 10

A mixture of 2-amino-4-chloro-7-methoxybenzothiazole (6.03 g), iodomethane (7 ml) in ethanol was heated under reflux for four days. The resulting mixture was then worked up in conventional manner to give 4-chloro-7-methoxy-3-methylbenzothiazolimine, m.p. 110.5-111.5°. (Compound 37)

Example 11

A mixture of compound 36 (6.03 g), acetic anhydride (10 ml) and pyridine (5 ml) was warmed for several minutes on a steam bath until most of the solid had dissolved. The mixture was then stirred at room temperature for 70 hours, treated with saturated aqueous sodium bicarbonate and stirred for a further hour. The resulting cream solid was collected and worked up in conventional manner to give N-acetyl-4-chloro-7-methoxy-3-methylbenzothiazolimine, m.p. 192.5-193.5°. (Compound 38)

Example 12

This example illustrates typical concentrates that can be formulated from compounds of the invention.

Wettable powder

| | |
|---|---|
| Compound of the invention | 25% w/w |
| Reax 45L* | 10% w/w |
| China clay | 65% w/w |

* = mixture of sodium lignosulphonate and an anionic wetting agent.

Granule

| | |
|---|---|
| Compound of the invention | 2% w/w |
| Gypsum granules | 98% w/w |

Test Example

The compounds of the invention were subjected to various tests.

a) Foliar tests

Compounds are assessed for activity against one or more of the following:

*Pyricularia oryzae*: rice blast (PO)

*Erysiphe graminis*: barley powdery mildew (EG)

*Plasmopara viticola*: vine downy mildew (PV)

*Phytophthora infestans*: late tomato blight (PI)

Aqueous solutions or dispersions of the compounds at the desired concentration, including a wetting agent, were sprayed onto the appropriate plant and then inoculated by spraying with spore suspensions of the fungi or by dusting or shaking diseased material over the treated plants for the *Erysiphe* spp.. Plants were then kept under controlled environment conditions suitable for maintaining plant growth and development of the disease. After an appropriate time, the degree of infection of the leaf surface was visually estimated.

Compounds were considered active if they gave greater than 50% control of the disease at a concentration of 125 ppm (w/v) or less.

Activities were demonstrated as follows (+ = active).

| Compound No | PO | EG | PI | PV |
|---|---|---|---|---|
| 1 | | | | |
| 2 | + | | | |
| 3 | + | | | |
| 4 | + | | | |
| 5 | | + | + | |
| 6 | + | | | + |
| 7 | + | | | |
| 8 | + | | | |
| 9 | + | | | |
| 10 | + | | | |
| 11 | + | | | |
| 12 | + | | | |
| 13 | + | | | |
| 14 | + | | | |
| 15 | + | | | |
| 16 | + | | | |
| 17 | | | | + |
| 18 | + | | | |
| 19 | + | | | |
| 20 | + | | | + |
| 21 | | | + | |
| 22 | + | | | |

| Compound No | PO | EG | PI | PV |
|---|---|---|---|---|
| 23 | + | + | | + |
| 24 | + | + | | |
| 25 | + | | | |
| 26 | | | | |
| 27 | | | | |
| 36 | + | | | |
| 37 | + | | | |
| 38 | + | | | |

## CLAIMS

1) A compound of formula I

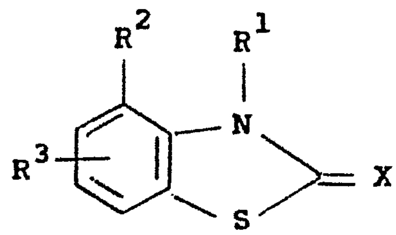

(I)

where $R^1$ is alkyl, alkenyl or alkynyl, each of which may be optionally substituted by halogen, alkoxy or aryl, $-N{=}CR^4R^5$ or $-NR^6R^7$;

$R^2$ is halo, hydroxy, amino, nitro, cyano, carboxy, alkoxycarbonyl or an optionally substituted alkyl, alkoxy, alkylthio, alkylamino, dialkylamino or alkylsulphonyl group;

$R^3$ is $-YR^9$;

$R^4$, $R^5$, $R^6$, $R^7$ and $R^8$ may be the same or different and are hydrogen, aryl or an optionally substituted alkyl, alkenyl or alkynyl group

$R^9$ has the same meaning as $R^8$ or is acyl;

X and Y are O, S or $NR^9$;

and when $R^1$ is $-N{=}CR^4R^5$ or $-NR^6R^7$, $R^3$ can also be hydrogen.

2) A compound according to claim 1 wherein $R^3$ is in the 7-position

3) A compound according to claim 1 or 2 wherein $R^1$ is alkyl, $R^2$ is halogen, $R^3$ is alkoxy, optionally substitued by alkoxy and X is oxygen.

4) A fungicidal composition comprising a compound as claimed in any one of the preceding claims in admixture with an agriculturally acceptable diluent or carrier.